# EUROPEAN PATENT APPLICATION

(11) **EP 2 239 251 A1**
(43) Date of publication of application: **13.10.2010**
(21) Application number: 09005158.2
(22) Date of filing: 08.04.2009
(51) Int. Cl.: C07C 303/44, C07C 309/14, A23K 1/16, A23L 1/03, A61K 31/185

(54) **Process for refining 2-aminoethanesulfonic acid from crude 2-aminoethane-sulfonic acid, 2-aminoethanesulfonic acid obtained there from and use thereof**

(71) Applicant: Rheln Pharma Consult GmbH, 40549 Düsseldorf (DE)
(72) Inventor: Yang, Chengxiong, 448000 Jingmen Stadt Hubei Provinz (CN)
(74) Representative: COHAUSZ DAWIDOWICZ HANNIG & SOZIEN

(57) **Abstract**

Disclosed is a process for refining 2-aminoethanesulfonic acid from crude 2-aminoethanesulfonic acid (content of at least 90%) obtained by chemical synthesis comprising the following steps:
i. heating a mixture of 100g of crude 2-aminoethanesulfonic acid dissolved in an adequate quantity of water, preferably between 230g and 250g, together with an effective amount of adsorbent to 85 °C to 95 °C
ii. adding 0.5g to 3g of an alcanol having 1 to 3 carbon atoms while changing the temperature to 88 °C to 92 °C for 10 to 120 min, preferably 20 to 40 min
iii. separating the adsorbent including impurities from the liquid
iv. crystallise 2-aminoethanesulfonic acid by controlled cooling of the mother liquid
v. separating the 2-aminoethanesulfonic acid crystals from the liquid and drying the same in a *per se* known manner

and use thereof.

## Description

The present Invention relates to a process for refining 2-aminoethanesulfonic acid from crude 2-aminoethanesulfonic acid, a high purity 2-aminoethanesulfonic acid obtained there from and use thereof.

### Background Art

Due to the limitation of source of raw materials, production cost and production capacity, chemical synthesis process is commonly used for 2-aminoothanesulfonic acid) biology extraction. There are approximately twenty ways of chemical synthesis to produce taurine depending upon the quality of raw material and processes efficiency. Among these 20 methods, ethylene oxide process is the most cost effective with highest yield-rate.

However, the process requires high temperature (260 °C to 270 °C) and high pressure (19MPa) to generate 2- aminoethane sodium (sodium Ox), and certain volume of liquid acid to be added in the reactor at fixed temperature (70 °C) and a pH of 7.5. After the neutralization, and centrifugal filters, 2-aminoethanesulfonic acid (taurine) is generated in the crude crystal form. In the crude taurine refined process, the current method use activated carbon for de-colorization (at 98 °C), followed by cooling the filtrate after filtration and the final step is crystallization of taurine precipitation and centrifugal separation.

2-Aminoethanesulfonic acid (taurine) is further obtained from isethionic acid (2-hydroxyethanesulfonic acid), which in turn is obtained from the reaction of ethylene oxide with aqueous sodium bisulfite. Another approach is the reaction of aziridine with sulfurous acid. This leads directly to 2-aminoethanesulfonic acid. Vakilwalla, M.V., Trivedi, D.M. In Curr Sci. 1949 Nov;18 (11):406 available under http://www.ncbi.nlm.nih.gov/pubmed/15398800 discloses a taurine production process having a purity of 80%.

CN 000100398096C = CN 1872029A of Yongan Pharma Corp. Ltd. discloses the preparation of spherical particles of taurine. This 2-aminoethanesulfonic acid prepared in accordance with the prior art has the crucial disadvantage that due to its low purity it tends to cake heavily after it is stored or used In compositions after a few days.

For that reason, an anti-caking agent such as silica must be always added to powders comprising 2-aminoethanesulfonic acid when used as a merchandise either in solid form or as a concentrate to be dissolved in a aqueous liquid In the food, cosmetic and drug industry.

### Summary of the Invention

The object of the present invention is to provide a process for refining 2-aminoethanesulfonic acid from a technical pure 2-aminoethanesulfonic acid which avoids e.g. this caking problem.

This object is solved by the measures disclosed In claim 1.

The invention thus provides a process for refining 2-aminoothanesulfonic add from crude 2-amlnoethanesulfonic acid (content of at least 90%) obtained by chemical synthesis comprising the following steps:
i. heating a mixture of 100g of crude 2-aminoethanesulfonic acid dissolved in an adequate quantity of water, preferably between 230g and 250g, together with an effective amount of adsorbent to 85 °C to 95 °C
ii. adding 0.5g to 3g, preferably 1g to 2 g, of an alcanol having 1 to 3 carbon atoms while changing the temperature to 88 °C to 92 °C for 10 to 120 min, preferably 20 to 40 min
iii. separating the adsorbent including impurities from the liquid
iv. crystallising 2-aminoethanesulfonic acid by controlled cooling of the mother liquid
v. separating the 2-aminoethanesulfonic acid crystals from the liquid and drying the same in a *per se* known manner.

The advantages of this invention are:

### 1. Higher taurine purity and energy saving:

Taurine purity has been improved by changing the crude taurine crystal and activated carbon de-colorization temperature to 90 °C, for better desalination and also reduces sulfate content In taurine significantly (from 0.8 per ten thousand down to the very extreme of 0.55 per ten thousand). Because of the lower temperature process, this invention also reduces energy consumption.

### 2. Quality improvement:

This optimal process design including taurine de-colorization by activated carbon, well calculated filtrate cooling and holding time (30 minutes cooling, and 30 minutes insulation), after mixing process in a mixer operated at the speed of 60 rev / min. A pure and beautiful cylindrical pin shape of taurine was produced with this unique process.

### 3. Increase taurine productivity:

Some certain amount of human consumable alcohol was added during taurine de-colorization at 90C, and taurine was dissolved completely, which Activated Carbon Absorption will function completely to absorb precipitated impurities thoroughly. The taurine crystal will become larger, and more pure (more than 99% purity).

### 4. Enlarge taurine crystal and improve the purity:

Add small amount of taurine crystal seeds (at 70 °C to 80 °C) during the crystallization process, will increase size and purity of taurine crystal.

### 5. Increase duration of taurine storage period:

After dry desiccant cooling (the temperature reached around 20 °C to 25 °C, relative humidity about 35%) taurine was packed with plastic-aluminium foil to prevent taurine to be moisturized and to be isolated from ambient temperature which will extend taurine storage period. (taurine will become stiff if moleturized).

In accordance with a preferred embodiment of the present invention the step (i) is performed in a closed system, preferably in an autoclave.

In accordance with a further preferred embodiment of the present invention the alcanol in step (ii) is added drop by drop or at a batch.

In accordance with a further preferred embodiment of the present invention the adsorbent is selected from the group consisting of activated carbon, alumina, silica gel and / or zeolite, preferably activated carbon

An alcanol having one to three carbon atoms is selected from the group consisting of methanol, ethanol, n-propanol and I-propanol. In accordance with a further preferred embodiment of the present invention the alcanol is selected from ethanol and / or I-propenol, preferably ethanol. The said ethanol has a purity of 95.6 % by volume to 100 % by volume, preferable 99 % by volume and being not denaturated for human use.

In accordance with a further preferred embodiment of the present invention step (iv) is performed in the presence of seed crystals, preferably 2-aminoethanesulfonic acid seeds. These seeds will be added in an amount of 50 ppm to 300 ppm, preferably 200 ppm.

In accordance with a further preferred embodiment of the present invention the controlled cooling is performed by first cooling down to 80 °C, adding the seeds, then cooling down to and holding 70 °C within 15 min to 45 min, preferably for 30 min, then cooling down and holding the temperature at 50 °C for 15 min to 45 min, preferably 30 min and then cooling down and holding the temperature at 25 °C for 15 min to 45 min, preferably 30 min.

In accordance with a further preferred embodiment of the present invention the cooling is performed while stirring the solution at a speed of 40 rev / min to 80 rev / min, preferably 60 rev / min.

In accordance with a further preferred embodiment of the present invention the separation in step (v) is made by a centrifugal.

In accordance with a further preferred embodiment of the present invention an amount of 0.05g to 2g, preferably 0.1g to 0.2g active carbon is employed as adsorbent.

The work-up including the drying of the crystals In step (v) is performed in a per so known manner by removing the rest of the water content using low pressure atmosphere and / or elevated temperature. Preferably dry boiling sterilization at a temperature of at least 100 °C is used.

The present invention further provides a high purity 2-aminoethanesulfonic acid.

Thus, provided is a 2-aminoethenesulfonic acid, obtainable according to the process presented therein before, having a sulfate content of less than 0.8 per ten thousand to 0.55 per ten thousand, preferably less than 0.7 per ten thousand to 0.55 per ten thousand measured per Chinese Pharmacopoeia, 2005, page 49, using colorimetry.

The present Invention finally provides several uses of the product obtainable by the process as presently called for.

First, the present invention provides biological compositions comprising 2-aminoethanesulfonic acid in high purity prepared according to the process presented therein before. These biological compositions are pharmaceutical and / or cosmetically compositions. These compositions could be applied either orally or topically.

Further, the present invention provides functional food and drinks comprising 2-aminoethanesulfonic acid in high purity according to the process presented therein before. The functional food comprises dairy products, grain products, foods for infants, bean products, and the functional drinks comprise energy drinks, both alcoholic and non alcoholic and hypotonic drinks.

Furthermore, the present invention provides animal food comprising 2-aminoethanesulfonic acid in high purity prepared according to the process presented therein before. For cats taurine is now a requirement of the Association of American Feed Control Officials (AAFCO) and any dry or wet food product labelled approved by the AAFCO should have a minimum of 0.1 % taurine in dry food and 0.2% in wet food. It is also a part of fish feeding products and of fed products of juvenile birds.

The present invention will now be explained in greater detail with reference to two figures.

The following is shown:
Fig. 1: A 2-aminoethanesulfonic acid powder obtained by a prior art process as described in the comparative example after a storage of 7 days.
Fig. 2: A 2-aminoethanesulfonic acid powder obtained by the process as claimed after storage of one month.

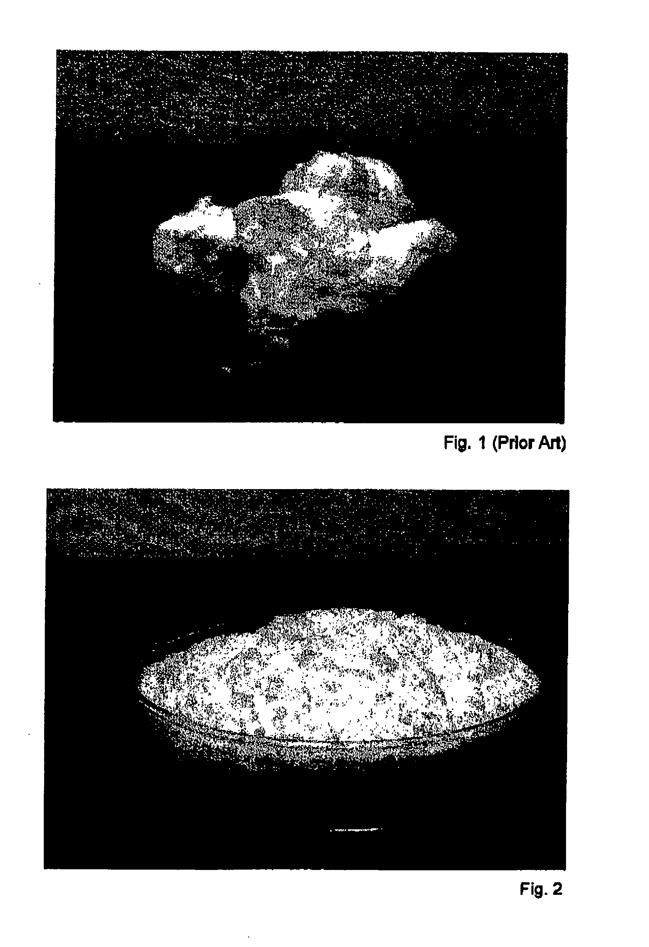

As illustrated in Fig. 1 the 2-aminoethanesulfonic acid powder is almost caked after a storage of 7 days. For commercial fluid compositions comprising 2-aminoethanesulfonic acid, e.g. provided as a powder, in capsules or as component of solutions such a caking should be avoided. This is not desired for use In commerce In fluid composition, such as powders and solutions.

In contrast thereto and as illustrated in Fig. 2 the 2-aminoethanesulfonic acid powder obtained by the process as claimed is not caked even after a storage of one month.

The present invention will now be Illustrated by a working example. In contrast thereto it is shown what properties are provided by a prior art process.

### Comparative Example (process of CN 187,202,917)

850kg 2-aminoethanesulfonic acid (obtained from Hanchuan Shi Zhongxin Chemical Product Ltd, Hanchan, Fenshul Zhen, product No PK205-008 having a taurine content of higher than 92%, 2400kg purified water and 3kg medical activated carbon were placed Into an autoclave, heating up to 98 °C, followed by de-colorization and pressure filtration to filter off activated carbon and other Impurities, The wet product was delivered to packing plant for crystallization of fine crystal autoclave treatment, cooling down to 20 °C to 25 °C, and then centrifuged with a centrifugal. The wet product weighted 500kg contents more than 93 percent taurine, with water at 3% - 7%, and placed In GFG efficient machine dry bollling sterilization, drying at 110 °C for 1 hour. At 35 °C, a mix materials or sub-screen process was conducted, and moved to quantitative packaging automated packaging line for packaging and ready to be shipped.

Fig 1 shows a sample of this solid product after a storage time of 7 days.

### Working Example

800kg crude 2-aminoethanesulfonic acid (obtained from Hanchuan Shi Zhongxin Chemical Product Ltd, Hanchan, Fenshul Zhen, product No PK205-008 having a taurine content higher than 90%), 2000kg of purified water and 1 kg of medical activated carbon were placed Into an autoclave, heated up to 93 °C. Then a certain amount of human consumable ethanol (1%) is added to the mixture when the temperature dropped down to 90 °C for 30min. During this time period de-colorization occurs and active carbon and other impurities were filtered off by pressure filtration.
The wet product was delivered to packing plant for crystallization of fine crystal autoclave treatment, add a certain amount of 2-aminoethanesulfonic acid seeds (200ppm) were added at 80 °C, then cooling down to 70 °C for 30min. to 50 °C for 30 min and to 25 °C for another 30min. Then the mixture was placed into a centrifuge to separate the crystals from the mother liquor. The wet solid crystal product is then placed Into an efficient machine for dry boiling sterilization, drying it at least at 100 °C.

2-Aminoethanesulfonic acid crystals are then placed into a plastic-aluminium foll bag after storing the dry 2-aminoethanesulfonic acid for cooling (20 °C to 25 °C) and de-moisturizing (30%).

Fig 2 shows a sample of this solid product after a storage time of 1 month..

### Content of sulphate impurities in 2-Aminoethanesulfonic acid

Further the sulphate content of the 2-Aminoethanesulfonic acid crystals was measured to find out the purity of the product an it was found to be less than 0.8 per ten thousand to 0.55 per ten thousand, preferably less than 0.7 per ten thousand to 0.55 per ten thousand, and especially at 0.55 per ten thousand. It was measured as follows:

Further it should be noted:
1. by changing the current taurine sulfate crystallization in the quality control methods, from the limits of their control (the 2005 edition of "Chinese Pharmacopoeia" was s 0.2%; JAPAN pharmacy (15 edition) standard was ≤ 0.010 %; the United States Pharmacopoeia (USP30) standard was ≤ 0.03%), should not know exactly in taurine content of sulphate ions. Through this research to Improve the sulfate ion content can accurately measure crystalline taurine sulfate ion content, on the optimization of the synthesis of taurine, as well as to improve the refining process, so that the purity of taurine crystal is higher, also increases the quality of taurine.
2. This is the first time, this method conducts a quantitative analysis of measuring the sulfate ion of taurine.

### 1. The original implementation of taurine:

1.1 The method of checking the limit of sulfate In taurine per the United States Pharmacopoeia: Add 0.8g goods to colorimetric Nessier's tube, add water to make dissolved into 40mL, diluted hydrochloric acid and then add 1mL, diluted with water to take Into a 50mL, as for test solution. 0.020N sulfuric acid solution 0.26mL to calorimetric Nessier's tube, add diluted hydrochloric acid 1mL, diluted with water to make into a 50mL, as control solution, 2mL barium chloride was added to the test solution and control solution, followed by both solutions being shaken, placed on a black background for 10 minutes then conduct a colour tube comparison by observation. Note: the test solution may not be more concentrated than the control solution by 0.03%.
1.2 The method of checking the limit of sulfate in taurine per Japanese medicine Bureau (15 edition): Add 2.0g goods into colorimetric Nessier's tube, dissolved into 40mL with water, and then add 1mL diluted hydrochloric acid, diluted again with water to make Into 50mL, as the test solution. Lessons 0.005mol / L sulfuric acid solution 0.40mL to colorimetric Nessler's tube, add diluted hydrochloric acid 1mL, diluted with water to make into a 50mL, as control solution followed by both solutions being shaken, placed on a black background for 10 minutes then conduct a colour tube comparison by observation. Note: the test solution may not be more concentrated than the control solution by 0.010%.
1.3 The method of checking the limit of sulfate in taurine per "Chinese Pharmacopoeia", 2005 edition, page 49, check 01.g goods, according to inspection procedure (Appendix VIII B), compared with standard potassium sulfate solution 2.0ml and shall not exceed 0.2% concentration of the control solution.
2. The proposed implementation modalities: determination of sulfate ion content in taurine crystal: Dissolve 10g taurine crystalline with water to 100ml, and then add 3ml barium chromate, 1ml calcium ammonia and 10ml of ethanol to 25ml test sample for filtering. A taurine sample will be become available through certain chemical process, and then we can see the wavelength through UV spectrophotometer to determine the content of the sample.

### Key points

1. Chromic acid barium suspension: first take 1g refined chromic acid barium, dissolved In 00ml acetic acid (1:35) and 100ml hydrochloric acid (1:50) mixture fully, shaken, and stored overnight

2. Take 1.4g calcium chloride, dissolved in 500ml of ammonia (1:4), the calcium-containlng clear liquid ammonia stored In polyethylene plastic bottle as standby.
Take 1.814g of potassium sulfate (110 ± 2 °C in the dry, 2 hour) exactly, add water dissolved into 1000ml measurement vessel, and add distilled water which will become standard solution of potassium sulfate. This standard solution will be diluted with water 10 times again when use.

3. Precisely draw the above standard solution of potassium sulfate 0.00, 1.00, 2.00, 3.00, 4.00, 5.00 respectively, to medium 50ml measurement vessel, diluted with water to 25ml, shake well, add 3ml of chromic acid barium suspension , shake for 2min, hold still for 5min, then while shaking add 1 ml clear liquid ammonia, calcium, 10ml 95% ethanol, add more water to the scale, shake for 1 min, stand still for 10 min, filter the solution, with 1cm colorimetric pool at scale of 380nm (or 2cm colorimetric pool, wavelength at scale of 20nm). Draw standard curve based on water as the control experiment, according to absorbency and sulfate contents.

4. Use 10g of taurine crystal sample and add water to 100ml, then pour 25ml Into a 50ml measurement vessel, add 3ml of chromic acid barium suspension, shake for 2 min, hold for 5min, and under shaking, add 1ml clear liquid ammonia, calcium, 10ml 95% ethanol, add water to 50ml, and shake for 1 min, stand still for 10min, filter solution, measure absorbency at 380nm with 1cm colorimetric pool, the measured absorbency, calculate the content of sulfate ions In taurine crystal based on sulfate content standard curve.

## Claims

1. A process for refining 2-aminoethanesulfonic acid from crude 2-aminoethanesulfonic acid (content of at least 90%) obtained by chemical synthesis comprising the following steps:
i. heating a mixture of 100g of crude 2-aminoethanesulfonic acid dissolved in an adequate quantity of water, preferably between 230g and 250g, together with an effective amount of adsorbent to 85 °C to 95 °C
ii. adding 0.5g to 3g, preferably 1g to 2g, of an alcanol having 1 to 3 carbon atoms while changing the temperature to 88 °C to 92 °C for 10 min to 120 min, preferably 20 min to 40 min
iii. separating the adsorbent including impurities from the liquid
iv. crystallising 2-aminoethanesulfonic acid by controlled cooling of the mother liquid
v. separating the 2-aminoethanesulfonic acid crystals from the liquid and drying the same in a *per se* known manner.

2. The process of Claim 1, **characterised in that** step (i) is performed in a closed system, preferably an autoclave.

3. The process of Claim 1, **characterised in that** the alcanol is added drop by drop or at a batch.

4. The process of Claim 1, **characterized in that** the adsorbent is selected from the group consisting of activated carbon, alumina, silica gel and / or zeolite, preferably activated carbon.

5. The process of Claim 1, **characterized in that** the alcanol is selected from ethanol and / or i-propanol, preferably ethanol.

6. The process of Claim 1, **characterized in that** step (lv) is performed in the presence of seed crystals, preferably 2-aminoethanesulfonic acid seeds.

7. The process in accordance with any of Claims 1 or 6, **characterized in that** the controlled cooling Is performed by first cooling down to 80 °C, adding the seeds, then cooling down to and holding 70 °C within 15 min to 45 min, preferably for 30 min, then cooling down and holding the temperature at 50 °C for 15min to 45 min, preferably 30 min and then cooling down and holding the temperature at 25 °C for 15 min to 45 min, preferably 30 min.

8. The process in accordance with any of Claims 1 or 7, **characterized in that** the cooling Is performed while stirring the solution at a speed of 40 rev / min to 80 rev / min, preferably 60 rev. / min.

9. The process of Claim 1 **characterized in that** the separation In step (v) is made by centrifugal.

10. The process of any of Claim 1 or 4, **characterized in that** an amount of 0.05g to 2g, preferably 0.1g to 0.2g active carbon is employed.

11. 2-Aminoethanesulfonic acid, obtainable according to any of Claims 1 to 10, having a sulfate content of less than 0.8 per ten thousand to 0.55 per ten thousand, preferably less than 0.7 per ten thousand to 0.56 per ten thousand measured per Chinese Pharmacopoeia, 2005, page 49, using colorimetry.

12. Biological compositions comprising 2-aminoethanesulfonic acid in accordance with Claim 11 or obtainable according to any of Claims 1 to 10.

13. Functional food and drinks comprising 2-aminoethanesulfonic acid in accordance with Claim 11 or obtainable according to any of Claims 1 to 10.

14. Animal food comprising 2-aminoethanesulfonic acid in accordance with Claim 11 or obtainable according to any of Claims 1 to 10.
